# EUROPÄISCHE PATENTSCHRIFT

(11) **EP 2 134 256 B1**
(45) Veröffentlichungstag und Bekanntmachung des Hinweises auf die Patenterteilung: **14.09.2011**
(21) Anmeldenummer: 08734358.8
(22) Anmeldetag: 07.03.2008
(51) Int. Cl.: A61B 5/083, A61B 5/087, A61M 16/20, G01N 33/00

(54) **TRANSPORTABLER PNEUMOTACHOGRAPH ZUR MESSUNG VON BESTANDTEILEN DES EXSPIRATIONSVOLUMENS SOWIE EIN VERFAHREN HIERZU**
PORTABLE PNEUMOTACHOGRAPH FOR MEASURING COMPONENTS OF AN EXPIRATION VOLUME, AND METHOD THEREFOR
PNEUMOTACHOGRAPHE TRANSPORTABLE POUR MESURER DES COMPOSANTS DU VOLUME D'EXPIRATION, ET PROCEDE CORRESPONDANT

(30) Priorität: 08.03.2007 DE 102007012210; 08.03.2007 DE 202007003818 U; 08.03.2007 DE 102007012285; 08.03.2007 DE 202007003817 U
(43) Veröffentlichungstag der Anmeldung: 23.12.2009
(73) Patentinhaber: AEROCRINE AB, 171 21 Solna (SE)
(72) Erfinder: EICHLER, Rüdiger, 97225 Zellingen (DE); DIETZE, Stefan, 16515 Lehnitz (DE); STEINHÄUSSER, Werner, 97268 Kirchheim (DE); BECHER, Gunther, 16321 Bernau (DE)
(74) Vertreter: Holmberg, Martin Tor
(86) Internationale Anmeldenummer: PCT/DE2008/000417
(87) Internationale Veröffentlichungsnummer: WO 2008/106961

(56) Entgegenhaltungen:
- US-A- 6 010 459
- US-A1- 2003 209 247
- US-A1- 2004 249 300
- US-B1- 6 425 393

## Beschreibung

Die Erfindung betrifft einen transportablen Pneumotachographen sowie ein Verfahren zur Messung von Bestandteilen, insbesondere von NO, des Exspirationsvolumens.

Stickoxide und andere gasförmige Verbindungen in der Ausatemluft werden zur Beurteilung des physischen Zustandes des Menschen herangezogen, denn sie sind Indikatoren für Stoffwechselprozesse im Organismus, Störungen und Erkrankungen des Menschen. Stationäre Geräte zur diagnostischen Gasanalyse der Ausatemluft sind bekannt und seit langer Zeit auf dem Markt erhältlich.

Ein transportabler Gasanalysator mit einem NO-Sensor ist in der EP 1 439 781 beschrieben, bei dem der Patient bei einer vorgeschriebenen Strömungsgeschwindigkeit und einem vorgeschriebenen Druck ausatmet. Nachteilig an dem dort beschriebenen Gerät ist, dass keine spirometrischen Messdaten erfasst werden können, die eine Korrelation von erfasstem Messwert zu dem entsprechenden erkrankten Lungenbereich ermöglichen.

Auch sind Geräte zur Lungenfunktionsanalyse, der Spirometrie seit Jahren in der Praxis in der Verwendung.

Die Spirometrie ist ein Verfahren zur Lungen-Funktionsprüfung. Dabei werden Lungen- und Atemvolumina gemessen und graphisch im Spirogramm dargestellt. Zur Erfassung der Lungenvolumina wird ein Spirometer oder auch Pneumotachograph benötigt.

Der Patient atmet über ein Mundstück in ein Atemrohr, wobei die Nase mit einer Nasenklemme verschlossen wird. Dabei misst das Spirometer elektronisch über einen Flusssensor die Luftströmungsgeschwindigkeit, mit der ein- und ausgeatmet wird, und daraus wird die Menge der geatmeten Luft pro Zeit berechnet. Die Luftmengen, die bei diesen Atemzügen bewegt werden, bildet das Gerät grafisch ab. So kann auch ein direkter Vergleich der Messwerte aus verschiedenen Tests erfolgen.

Durch Messung der Luftströmungsgeschwindigkeit oder Ausatemgeschwindigkeiten und der Lungenvolumina ist es dem Arzt möglich, Erkrankungen der Lunge zu diagnostizieren und in ihrem Verlauf zu kontrollieren. Folgende Werte können mit Hilfe der Spirometrie gemessen werden:
Atemzugvolumen (AZV): Es entspricht dem ein- bzw. ausgeatmeten Volumen bei normalem Atemzug.
Inspiratorisches Reservevolumen (IRV): Dies ist das Volumen, das nach normaler Einatmung noch zusätzlich eingeatmet werden kann.
Expiratorisches Reservevolumen (ERV): Es ist das Volumen, das nach normaler Ausatmung noch zusätzlich ausgeatmet werden kann.
Inspiratorische Kapazität (IC): Sie ist definiert als das Volumen, das nach normalem Ausatmen maximal eingeatmet werden kann.
Vitalkapazität (VC) ist das Volumen, das nach maximaler Einatmung maximal ausgeatmet werden kann. Einsekundenkapazität (FEV1, Tiffeneau-Test) ist das Volumen, das bei maximaler Einatmung in einer Sekunde maximal ausgeatmet werden kann.

Diese Messgrößen helfen beispielsweise zwischen den beiden Hauptgruppen von Luhgenerkrankungen zu unterscheiden:
Obstruktive Lungenerkrankungen: Sie werden durch eine Verengung der Atemwege verursacht, z.B. durch Asthma oder COPD.
Restriktive Lungenerkrankungen: Dabei sind Lunge und/oder Brustkorb vermindert dehnbar. Beispiele sind Lungenverhärtung (Lungenfibrose), Flüssigkeitsansammlung im Lungenspalt (Pleuraerguss) oder ein hoch stehendes Zwerchfell (Zwerchfellparese).

Bei der Spirometrie atmet der Patient über ein Mundstück ein bzw. aus. Das Mundstück ist mit einem Spirometer verbunden und in den meisten Fällen mit einem Bakterienfilter versehen. Zur Erfassung der verschiedenen Messgrößen muss der Patient die Anweisungen des Untersuchenden bezüglich Ein- und Ausatmung genau befolgen. Sonst werden falsche Werte gemessen, die wiederum zu falschen Rückschlüssen bei der Behandlung führen können. Die Untersuchung hängt somit von einer gute Mitarbeit des Patienten ab.

Ein weiteres Gerät zur Messung von NO im Ausatemvolumen ist in der US-Schrift 6 010 459 beschrieben. Hier erfolgt eine Spirometrie nach der Messwerterfassung. Der Patient atmet dort synthetische mit NO versetzte und nachträglich befeuchtete Luft ein. Bei Ausatmen muss der Patient einen definierten Druck im Messgerät erzeugen, vorgeblich, damit sich das Gaumen-Rachensegel beim Ausatmen schließt und keine, die Messung verfälschende Luft des Nasen-Rachenraums in den Ausatemstrom gerät, in welchem die NO-Konzentration das bis zu 100 fache im Vergleich zur Lungenausatemluft betragen kann. Nachteilig an diesem Gerät ist die Ortsgebundenheit, da zur Messung das synthetische Gas zum Einatmen verwendet werden muss.

Zudem liegt mit den 2005 veröffentlichten Guidelines der ATS/ERS (Exhaled breath condensate: methodological recommendations and unresolved questions. I. Horvath, J. Hunt and P.J. Barnes, On behalf of the ATS/ERS Task Force on Exhaled Breath Condensate, Eur Respir J 2005; 26: 523 - 548) erstmals eine Gesamtdarstellung der Methoden zur Atemkondensatdiagnostik vor. Guidelines zur NO-Messung sind in den Schriften: "ATS Workshop Proceedings: Exhaled Nitric Oxide and Nitric Oxide Oxidative Metabolism in Exhaled Breath Condensate: Executive Summary. Am J Respir Crit Care Med. 2006 Apr 1; 173(7) : 811-813" und "American Thoracic Society Documents: ATS Workshop Proceedings: Exhaled Nitric Oxide and Nitric Oxide Oxidative Metabolism in Exhaled Breath Condensate. Proc Am Thorac Soc Vol 3. pp 131-145, 2006" dargelegt.

Das Dokument US 2004/0249300 offenbart einen Transportablen Pneumotachographen aufweisend einen Prozessor, einen einatemseitig am Pneumotachographen angebrachten Filter, einen im oder am Pneumotachographenrohr angebrachten Sensor, wobei das Pneumotachographenrohr sensorseitig eine Öffnung aufweist und / oder mindestens ein im Pneumotachographenrohr eingerichtetes Mittel zur Probennahme.

Das Dockument US 2003/0209247 offenbart ein PEEP Ventil für die Aufrechterhaltung eines positiven Drucks im Atemweg eines Patienten.

Es ist zu konstatieren, dass die Standardisierung der Probennahme, Probenlagerung und Analytik noch zu verbessern ist. Die Standardisierung der Probennahme muß zukünftig ebenfalls erfolgen. Speziell bei der Analytik ist daher den verwendeten Verfahren und deren Validierung mehr Aufmerksamkeit zu schenken.

Der Erfindung liegt daher das technische Problem zu Grunde, einen Pneumotachographen zum Messen von Bestandteilen des Ausatemvolumens anzugeben, der transportabel und handlich ist und eine Korrelation von erfassten Messdaten eines oder mehrerer Bestandteile des Ausatemstromes mit einer Lungenfunktionsprüfung erlaubt, wobei die Analytik unter Beachtung standardisierender Guidelines erfolgen kann und eine Lokalisierung des Krankheitsherdes ermöglicht.

Gelöst wird das technische Problem, indem die Erfindung einen transportablen Pneumotachographen zur Bestimmung von Bestandteilen des Exspirationsvolumens angibt, der einen Prozessor, ein ausatemseitig am Pneumotachographen angebrachtes PEEP-Ventil, einen einatemseitig am Pneumotachographen angebrachten Filter zum Entfernen des Anteils an zu bestimmendem Bestandteil in der Inspirationsluft, mindestens einen im oder am Pneumotachographenrohr angebrachtem Sensor, wobei das Pneumotachographenrohr sensorseitig eine Öffnung aufweist und/oder mindestens ein im Pneumotachographenrohr eingerichtetem Mittel zur Probennahme und eine optische und/oder akustische Kontrolle des Exspirationsstroms aufweist.

Kernstück des transportablen Pneumotachographen zur Bestimmung von Bestandteilen des Exspirationsvolumens ist ein Pneumotachograph, vorzugsweise mit einem austauschbaren Mundstück, in dem ein Bakterienfilter eingesetzt ist. Durch den Pneumotachographen atmet der Patient ein und aus. Einatemseitig ist am Pneumotachographen ein Filter zum Herausfiltern des zu messenden Bestandteils aus der Umgebungsluft vorgesetzt. Im Pneumotachographen ist in Strömungsrichtung der Ausatmung hinter denen im Pneumotachographenrohr zur Erzeugung eines Strömungswiderstandes befindlichen Lamellen oder Gitter im Rohr, beispielsweise in der Strömungsmitte, oder in der Rohrwand ein Sensor und/oder eine Probenahme, beispielsweise in Form einer Kanüle, die vorzugsweise in den Strömungskanal des Pneumotachographenrohres hineinragt, eingerichtet. Das bedeutet, dass bei der ersten Ausführung der Sensor oder die Sensoren oder deren empfindliche Schicht sich direkt im Mainstream befinden und vorzugsweise ein Anschalten und/oder Freigeben des Sensors zum gewünschten Messzeitpunkt erfolgt. Zum Schutz vor Verschmutzung durch Speichel oder andere Stoffe ist der Sensor oder die Probenahmeöffnung in Strömungsrichtung des Ausatemstromes ausgerichtet sein und so geformt, dass kein Speichel und kein möglicherweise entstehendes Kondenswasser in die Probenahmeöffnung hineingelangen kann. Die Ausatmung erfolgt gegen einen exspiratorischen Widerstand, der durch das ausatemseitig am Pneumotachographenrohr angebrachte PEEP-Ventile erzeugt wird. Der exspiratorische Widerstand beträgt vorzugsweise 5 bis 20 cm H₂O und bedingt, dass sich der Atemwegsmitteldruck und die funktionelle Residualkapazität erhöht.

Mittels des PEEP-Ventils wird erreicht, dass die Entlüftung der Lunge bzw. Lungenbläschen auch bei einer Atemwegsverengung, Sekret in den Atemwegen oder andersartigen Belüftungsstörungen (Verteilungsstörung) gleichmäßiger erfolgt. Diese Maßnahme ist Voraussetzung für eine reproduzierbare Wiederholung der Atemmanöver und einer weitgehend ungestörten Emission der Bestandteile der Ausatemluft während des Messvorganges.

Um die Gefahr einer Fehlmessung durch Beimischung von Nasenluft zu vermindern können folgende Maßnahmen vorgesehen sein:
- Tragen einer Nasenklammer bei der Inspiration
- Ausatmung mit konstantem Flow bzw. Flow größer Null, denn ein Stopp des Flow während des Ausatemmanövers bringt Luft aus der Nase in den Rachen.

Ein konstanter Flow ist ein Flow mit einer maximalen Abweichung von +/- 10% vom Mittelwert.

Zur Kontrolle des Flows weist der transportable Pneumotachograph eine optische oder akustische Kontrolle auf, anhand derer der Patient seine Ausatmung kontrollieren und einstellen kann.

In einer bevorzugten Ausführungsform ist zwischen dem einatemseitig am Pneumotachographen angebrachten Filter, der austauschbar eingerichtet ist, und dem Pneumotachographenrohr ein Ventil oder eine Klappe eingerichtet, welches bei der Exspiration die Eintrittsöffnung des Pneumotachographen verschließt. Hiermit wird erreicht, dass der Patient nur gegen den durch das PEEP-Ventil exspiratorischen Widerstand atmet. Vorzugsweise schließt das Ventil oder die Klappe selbsttätig. Ein solches Ventil kann ein Rückschlagventil, ein Sperrventil oder eine einfache Klappe, die sich in das Pneumotachographenrohr öffnet, sein. Aber es sind hier keine Begrenzungen bauteiletechnischer Art vorgesehen.

Der Sensor kann aus der Gruppe "elektrochemischer Sensor, Chemilumineszenssensor, NO-Sensor, O₂-Sensor, H₂O₂-Sensor, CO₂-Sensor, CO-Sensor" und/oder einen Kombinationssensor aus den genannten Sensoren ausgewählt sein. Je nach zu analysierendem Bestandteil (NO, O₂, ...) der Ausatemluft ist der entsprechende Sensor einsetzbar. Es können auch mehrere Sensoren zum gleichzeitigen Messen verschiedener Bestandteile eingerichtet sein.

In der Öffnung zwischen Sensor und Pneumotachographenrohr kann ein regelbares und/oder ansteuerbares Ventil, im weiteren sensorisches Ventil genannt, oder eine regelbare oder ansteuerbare Pumpe, im weiteren sensorische Pumpe genannt, eingerichtet sein. Hierzu kann der Sensor außerhalb des Pneumotachographenrohres eingerichtet und mit dem Pneumotachographenrohr über eine Leitung und eine Probenahme verbunden sein. Das sensorische Ventil oder die sensorische Pumpe können nach der Maßgabe des das Pneumotachographenrohr passierenden Inspirations- oder Exspirations- oder eines Teilinspirations- oder Teilexspirationsvolumens oder -stromes den Zustand "offen" oder "geschlossen" haben, wobei vorzugsweise das sensorische Ventil durch den Prozessor ansteuerbar ist. Hiermit wird erreicht, dass eine Probennahme bzw. Messung nur dann durchgeführt wird, wenn definierte Volumenströme oder Teilvolumenströme der Exspiration das Pneumotachographenrohr passieren. Der Zeitpunkt, wann eine Messung zu erfolgen hat, wird über die vom Pneumotachographen ermittelte Fluß-Volumen-Korrelation berechnet, welcher mit einem Prozessor verbunden ist, an welchem auch das sensorische Ventil angeschlossen und von diesem ansteuerbar ist. Da die Probennahme prozessorgesteuert direkt aus dem Pneumotachographen erfolgt, wird jede Zeitverzögerung oder Diskordanz zwischen Flowmessung und Probennahme primär ausgeschlossen

Die optische Kontrolle des Exspirationsstroms kann aus der Gruppe "y-t Graph, Balkengraphik, Leuchtdiodenanzeige mit einer oder mehreren Leuchtdioden" ausgewählt sein. Die akustische Kontrolle kann ein Piepton oder ein sich in der Lautstärke oder Frequenz ändernder Ton sein.

In einer besonderen Ausführungsform ist das PEEP-Ventil ein Doppelventil, welches den Flow zwischen einem Mindestflow und einem Maximalflow begrenzt. Anders ausgedrückt: Das Ventil öffnet bei Erreichen eines zum Mindestflow in Korrelation stehenden ersten Ausatemdruck, wodurch die Strömungsgeschwindigkeit im Pneumotachographenrohr sprunghaft vom Wert "Null" auf die Strömungsgeschwindigkeit des Mindestflow ansteigt. Der Patient kann nun bei einem definierten Flow ausatmen. Bei Überschreiten eines zweiten gegenüber dem ersten Ausatemdruck größeren zweiten Ausatemdruck, der mit dem Maximalflow korreliert, schließt das Ventil wieder und der Wert der Strömungsgeschwindigkeit fällt sprunghaft auf den Wert "Null". Der Patient ist nun an der Ausatmung gehindert. Ein Ausatmen ist nur zwischen dem Mindestflow und dem Maximalflow möglich. Ein solches PEEP-Doppelventil weist beispielsweise eingangsseitig (pneumotachographenseitig) ein federbelastetes Rückschlagventil und ausgangsseitig (Umgebung) ein Druckventil auf, wobei das federbelastete Rückschlagventil erst bei Erreichen bzw. Überschreiten eines eingangsseitig erzeugten ersten Druckes öffnet und das Druckventil ausgangsseitig bei Überschreiten eines eingangsseitig erzeugten höheren Staudruckes, der größer als der erste Druck (Öffnungsdruck) ist, schließt. In anderen Worten ausgedrückt: Das PEEP-Ventil öffnet an seiner Eingangsöffnung erst ab Überwindung eines ersten definierten Druckswiderstandes. Ab diesen Zeitpunkt ist dessen Ausgangsöffnung geöffnet. Bei Steigerung des Flows erhöht sich gleichzeitig der Druck auf das Rückschlagventil und die Rückschlagsfeder wird weiter zusammengedrückt und drückt gleichzeitig in Richtung der Ausgangsöffnung. Rückseitig zwischen Verschluß des Rückschlagsventils und der Feder ist beispielsweise ein Kegel angebracht, der beim Zusammendrücken der Feder in die Ausgangsöffnung einfasst und diese sukzessive verschließt. Mit dieser Art PEEP-Ventil kann der exspiratorische Flow und der exspiratorische Widerstand zwischen einem Mindestwert und einem Maximalwert, die jeweils einstellbar sind, begrenzt werden.

Der transportable Pneumotachograph kann einen oder mehrere gasundurchlässige Sammelbehälter zum Auffangen von Proben und/oder von Atemvolumina mehrerer Atemzüge aufweisen. Mit diesen können mehrere Atemzüge vereinigt oder Proben bis zur Analyse aufbewahrt werden.

Die Oberflächen der probeführenden Leitungen oder der Luftführungskanäle des erfindungsgemäßen Pneumotachographen können modifiziert und dergestalt sein, dass auf diesen Membranen, flüssige Filme aufgetragen oder Einlage von porösen Schichten oder Membranen eingearbeitet sind, so dass bestimmte Bestandteile der Probengase zurückgehalten bzw. in den Schichten chemisch gebunden werden. Die Aufzählung ist beispielhaft und nicht begrenzend. So können beispielsweise der Wasserdampfanteil der Probe durch hygroskopische Substanzen oder wasserbindende Schichten vermindert werden oder die Analyse störende Substanzen bzw. Chemikalien aus dem Ausatemstrom oder Einatemstrom physikalisch oder chemikalisch gebunden werden, um eine störungsfreie Analyse gewährleisten zu können.

Die Abscheidung von Wasserdampf auf dem oder den Sensoren und/oder in den Zuleitungen zu dem oder den Sensoren kann auch durch Temperierung auf eine der Ausatemluft entsprechende Temperatur von 35 bis 40 °C verhindert werden.

Andererseits können freie Radikale durch chemische Umsetzung von in oder an den modifizierten Oberflächen deaktiviert und so die Probe stabilisiert werden. Des Weiteren können Bestandteile des Ausatemstromes, die in einer nicht analysierbaren oder durch Kreuzreaktionen überlagerten Form vorliegen in eine selektiv nachweisbare Form überführt werden. Die Modifizierung der Oberflächen der probeführenden Leitungen oder der Luftführungskanäle kann auch ein biologisches Immobilisat, beispielsweise ein immobilisiertes Enzym sein, das organische Verbindungen spezifisch umsetzt oder Bestandteile spezifisch bindet.

Der transportable Pneumotachograph kann zusätzlich mit einer Spüleinrichtung ausgestattet sein, die es erlaubt, dass der Sensor und /oder die Probenahme mit einem Gas ausgewählt aus der Gruppe "Bestandteilsfreie Luft, synthetische Luft, zum Zwecke der Kalibrierung hergerichteten Gase oder einer Kombination der genannten Gase" gespült und damit von der Ausatemluft gereinigt wird, um die Genauigkeit der Messungen zu erhöhen. Hierzu kann eine Pumpe eingerichtet sein, die beispielsweise mit dem einatemseitig am Pneumotachographen angebrachten Filter über eine, vorzugsweise separate Leitung verbunden ist und Umgebungsluft durch den Filter in den Probennahme- oder Sensorbereich pumpt. Die Spülluft kann über das PEEP-Ventil an die Umgebung oder über eine separate Spülgasleitung entsorgt werden. Es kann aber auch ein oder mehrere Anschlüsse zum Anschließen einer unter Druck stehenden oder druckfreien Spülgasflasche eingerichtet sein.

In einer weiteren Ausführungsform ist eine Kalibrierung des Sensors mit einem Gas, ausgewählt aus der Gruppe "Bestandteilsfreie Luft, synthetische, zum Zwecke der Kalibrierung hergerichteten Gase oder einer Kombination der genannten Gase" eingerichtet. Die Kalibrierwerte der genannten Gase sind individuell am Gerät einstellbar.

Des Weiteren lehrt die Erfindung ein Verfahren zur Bestimmung von Bestandteilen des Exspirationsvolumens aufweisend die Verfahrensschritte:
a) Filtern des zu bestimmenden Bestandteils oder der zu bestimmenden Bestandteilen aus der eingeatmeten Luft
b) Messen des Einatemvolumens
c) Ausbildung eines positiven Druck in der Lunge gegen ein durch geschlossenes Druckventil erzeugten Druckwiderstand
d) Öffnen des Druckventil durch Überwinden des Druckwiderstandes
e) Ausbildung eines definierten exspiratorischen Flows auf einem Druckniveau höher als der vorgegebene Druckwiderstand
f) Verwerfen der Totraumluft des Mund- und Rachenraums,
g) Probenahme der exspiratorischen Luft und/oder sensorische Messung des zu bestimmenden oder der zu bestimmenden Bestandteile in der exspiratorischen Luft nach Verwerfen der Totraumluft.

In der Praxis sieht die Verfahrensweise dann, beispielsweise bei einer NO-Analyse, wie folgt aus: Der Patient wird aufgefordert, tief auszuatmen. Sofort danach muss der Patient über ein Filtermundstück durch den erfinderischen Pneumotachographen tief einatmen. Diese Einatmung erfolgt beispielsweise durch einen Aktivkohle-Luftfilter (Atemfilter) durch den Pneumotachographen in die Lunge.

Dieser Atemzug wird registriert und das zu erwartende Ausatemvolumen anhand des Einatemvolumens berechnet. Danach muss der Patient ohne vom Mundstück abzugehen langsam tief ausatmen.

Dieser Atemzug soll, vom Analysator vorgegeben, gegen einen exspiratorischen Widerstand, vorzugsweise im Bereich von 5 bis 20 cm H₂O, induziert durch das PEEP-Ventil erfolgen. Mit einem Kontrollsystem (optische oder akustische Kontrolle) wird dem Patienten dabei der exspiratorische Flow angezeigt, vorzugsweise als y-t-Graph auf einem Screen. Ersatzweise kann hier eine Balkengraphik oder verschiedenfarbige Leuchtdioden zur Anwendung kommen. Die akustische Kontrolle kann ein Piepton oder ein in der Lautstärke oder Frequenz sich ändernder Ton sein.

Der exspiratorische Fluss soll vorzugsweise 50 ml/sec betragen. Dieser kann aber auch variiert werden. Der Flow von 50 ml/sec sollte in einem Bereich von +/- 10 % für 4 sec bei Kindern unter 12 Jahren oder 6 sec bei Kindern über 12 und Erwachsenen gehalten werden. Das entspricht bei einem Flow von 50 ml/sec etwa 300 ml Luft insgesamt.

Die Anpassung an die für die Analyse eines oder mehrerer Bestandteil der Ausatemluft benötigten Flowraten und Drücke beispielsweise aufgrund geforderter Rahmenbedingungen durch gesetzliche Bestimmungen oder Richtlinien erfolgt durch Auswahl und Einsatz eines den geforderten Flow und Druck maßgebendes PEEP-Ventil oder bei einem regelbaren PEEP-Ventil durch Einstellung dessen an die geforderten Parameter.

Während dieses Plateau sollte der NO-Messwert in einem Bereich von +/- 10 % bleiben.

Die Messung ist zu wiederholen. Eine Messung unterliegt den ATS/ERS-Richtlinien, wenn mindestens zwei Atemmanöver den Kriterien entsprechen.

Ein weiterer Verfahrensschritt kann sein, dass ein Teil- und/oder das Gesamtexspirationsvolumen in einem gasundurchlässigen Auffangbehältnis, vorzugsweise einem Gasbeutel, aufgefangen wird.

Die Probennahme und/oder der Sensor kann des Weiteren mit einem oder mehreren Gasen ausgewählt aus der Gruppe "Bestandteilsfreie Luft, synthetische, zum Zwecke der Kalibrierung hergerichteten Gase oder einer Kombination der genannten Gase" nach jeder Messeinheit gespült werden.

Eine Kalibrierung des Sensors kann mit einem oder mehreren Gasen ausgewählt aus der Gruppe "Bestandteilsfreie Luft, synthetische, zum Zwecke der Kalibrierung hergerichteten Gase oder einer Kombination der genannten Gase" nach einer oder einer Anzahl von Messeinheiten erfolgen.

Die bestandteilsfreie Luft kann erzeugt werden, indem Raumluft einatemseitig durch den am Pneumotachographen angebrachten Filter gepumpt wird. Diese gereinigte Luft wird anschließend über den Sensor geleitet.

Der konstante Flow der exspiratorischen Luft kann vorzugsweise 10 - 500 ml/s, insbesondere 45 bis 55 ml/s betragen.

Der exspiratorische Flow muss für eine Dauer von 1 bis 30 s, vorzugsweise von 2 bis 10 s, insbesondere von 4 bis 6 s, konstant gehalten werden.

Es kann anhand des Wertes des Einatemvolumens und einer entsprechenden mathematischen Korrelation zum zu erwartenden Ausatemvolumen eine Zuordnung der Messwerte zu zeitlich nacheinander ausgeatmeten Teilvolumenströme eines Exspirationsvolumens erfolgen, wobei diesen Teilvolumenströme bestimmten Regionen und Zonen des Respirationstraktes zugewiesen werden können. Durch die Operation können Erkrankungen und Störungen des Respirationstraktes lokalisiert werden.

Ein weiterer Verfahrensschritt sieht vor, dass eine Messung von Bestandteilen des Exspirationsvolumens nur dann erfolgt, wenn ein definiertes Teilexspirationsvolumen den Sensor passiert. Durch die spirometrische Messdatenerfassung kann über den Prozess berechnet werden, zu welchem Zeitpunkt ein Teilvolumenstrom x, der aus einem Bereich Y der Lunge stammt, in dem beispielsweise ein Krankheitsherd vermutet wird, den Sensor passiert. Eine Messung der Bestandteile nur dieses Teilvolumenstroms kann dann gestartet werden.

Unter den Bedingungen von Standards, beispielsweise der ATS/ERS-Richtlinien, sind nur Messwerte auswertbar und repräsentativ, die unter definierten Messbedingungen gewonnen wurden. Hierzu sieht das erfinderische Verfahren vor, dass eine Messung von Bestandteilen des Exspirationsvolumens nur bei Eintreten des oder der vorgegebenen Parameter des Exspirationsvolumens "Überwinden des exspiratorischen Widerstands" und/oder "konstanter exspiratorischen Flow" und/oder "Dauer des exspiratorischen Flows" erfolgt. Werden diese Parameter nicht erreicht oder nicht lange genug gehalten, wird kein Messwert aufgenommen, d.h. der Sensor bekommt kein Signal vom Prozessor, eine Messung einzuleiten. Insofern keine Steuerung des Sensors erfolgt oder ein Bedingung nach Einleitung der sensorischen Messung nicht eingehalten wurde, kann ein Messwert, der unter Nicht-Standard-Bedingungen ermittelt wurde, als ein solcher gekennzeichnet und ausgegeben werden.

Vorzugsweise sind die Werte der Parameter "Überwinden des exspiratorischen Widerstands" und/oder "konstanter exspiratorischen Flow" und/oder "Dauer des exspiratorischen Flows" individuell einstellbar und/oder diese in Abhängigkeit von der Patientengruppe und/oder dem Zustand des Exspirationstraktes des Patienten aus einem Speichermedium, vorzugsweise dem Prozessor, abrufbar.

In einer besonderen Ausführungsform ist das PEEP-Ventil abnehmbar eingerichtet. Hierdurch kann der transportable Pneumotachograph auch zur Messung von spirometrischen Daten verwendet werden, ohne das ein weiteres Spirometer bereitgestellt werden muss. Für die Zeit der Aufnahme der spirometrischen Messdaten ist der Messsensor vorzugsweise außer Betrieb gesetzt oder abgeschaltet.

Nachstehend wird die Erfindung anhand von Ausführungsbeispielen näher erläutert. Es zeigen:
- **Fig.1:**: schematische Darstellung des transportablen Pneumotachographen,
- **Fig. 2:**: ein Peep-Ventil und
- **Fig. 3:**: Kennlinien des Peep-Ventils

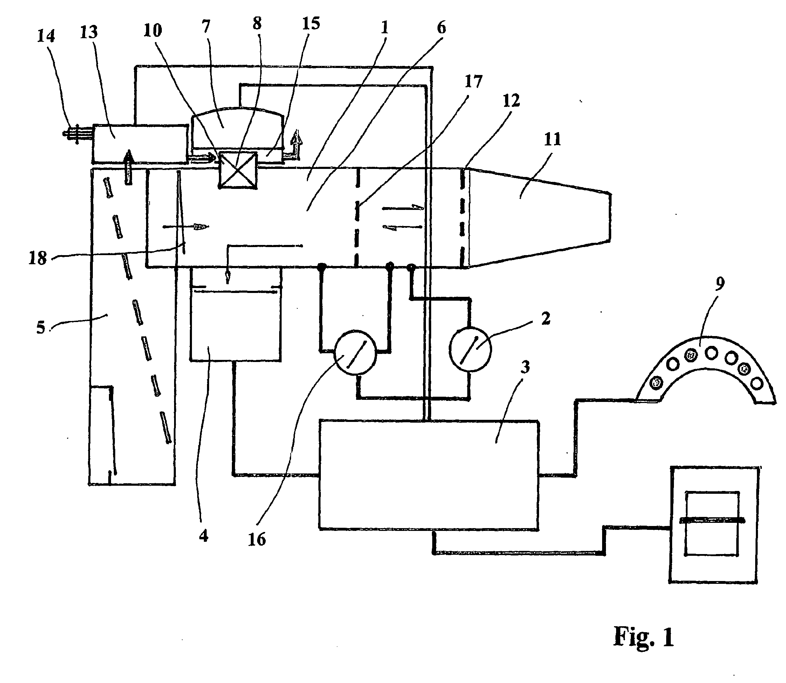

Die **Fig. 1** zeigt einen transportablen Pneumotachographen zur Bestimmung von Bestandteilen des Exspirationsvolumen mit einem Pneumotachographen 1 mit einem Mittel zur Druckmessung 2 und einem Prozessor 3, einem ausatemseitig am Pneumotachographen 1 angebrachtes PEEP-Ventil 4, einem einatemseitig am Pneumotachographen 1 angebrachten Filter 5 zum Entfernen des Anteils an zu bestimmendem Bestandteil in der Inspirationsluft, einem am Pneumotachographenrohr 6 angebrachtem Sensor 7, wobei das Pneumotachographenrohr 6 sensorseitig eine Öffnung 8, in der ein Flowcontroller 10 eingerichtet ist, aufweist, und einer optische Kontrolle 9 des Exspirationsstroms. Das Mundstück 11 ist mit einem Bakterienfilter 12 ausgestattet. Zum Spülen des Sensors 7 pumpt die Pumpe 13 (mit einem zusätzlichen Anschluss 14 zum Anschließen eines Spülgasbehälters) das Spülegas, hier vom zu bestimmenden Bestandteil gereinigte Umgebungsluft, durch den Filter 5 in die Sensorkammer 15. Der Pneumotachograph weist standardgemäß ein elektrisches Manometer 16 auf, welches den Druckunterschied vor und hinter den Lamellen 17 misst.

Zwischen dem einatemseitig am Pneumotachographen 1 angebrachten Filter 5 und dem Pneumotachographenrohr 6 ist eine inspiratorisch wirksame Klappe 18 eingerichtet, welches bei der Exspiration die Eintrittsöffnung des Pneumotachographen verschließt.

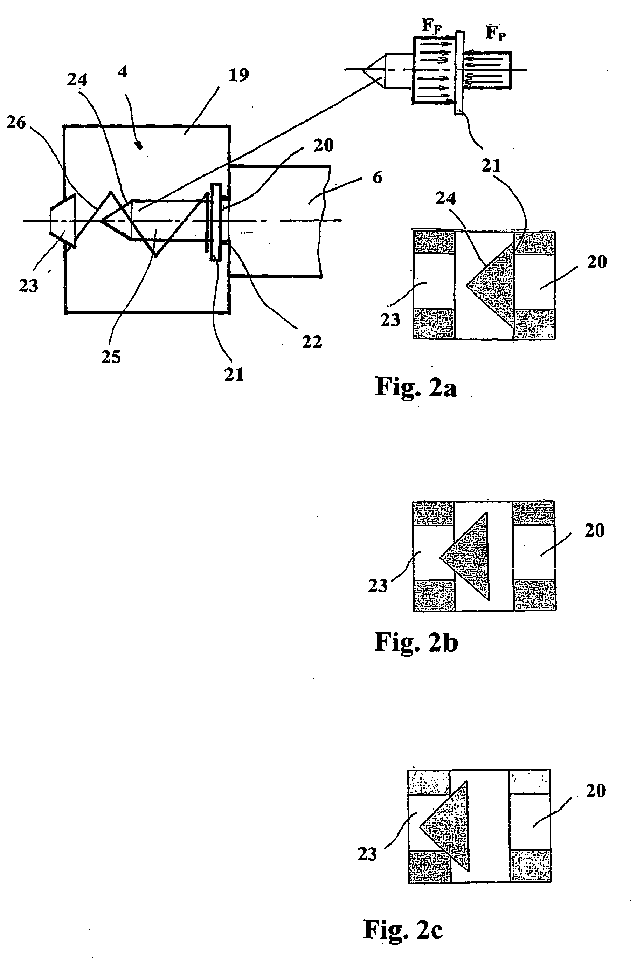

Das in den **Fig. 2a** **- c** dargestellt PEEP-Ventil 4 besteht aus einem Gehäuse 19 mit einer Einströmöffnung 20 vom Pneumotachographenrohr 6 und einer Ausströmöffnung 23 sowie dem innerhalb des Gehäuses 19 vor der Einströmöffnung 20 angeordneten, ein Rückschlagventil bildenden Ventilteller 21 mit Ventilsitz 22 an der Einströmöffnung 20. Der Ventilteller 21 wird durch die Druckfeder 26, die sich an der der Einströmöffnung gegenüberliegenden Gehäusewand abstützt, gegen den Ventilsitz 22 gedrückt und dichtet so die Einströmöffnung 20 ab. Erreicht die durch den PEEP im Pneumotachographenrohr 6 erzeugt Kraft F_{P} einen größeren Wert als die Federkraft F_{F}, öffnet der Ventilteller 21 die Einströmöffnung 20 und der Flow kann vom Pneumotachographenrohr 6 gegen den Widerstand des PEEP-Ventils 4 diesen durchströmen und über die Ausströmöffnung 23.

Erhöht sich der Flow und damit der PEEP und damit die Kraft F_{P} verschiebt sich der Ventilteller 21 weiter weg vom Ventilsitz 22, bis der auf der Rückseite des Ventiltellers 21 angeordnete Verschluss 24, der zusammen mit der Ausströmöffnung 23 ein Druckventil bildet, diese verschließt. Damit nimmt der Flow den Wert 0 an, d.h., es kann vom Patienten in das Pneumotachographenrohr 6 keine Luft mehr oder nur in einer noch geringen Menge unter großem Druck eingeblasen werden.

Durch die Anordnung des Verschlusses 24 über einen Schaft 25 am Ventilteller 21 und die Möglichkeit einer Einstellung der Schaftlänge, besteht ein funktionaler Zusammenhang zwischen PEEP und Verschließen der Ausströmöffnung 23, so dass sich Grenzen für einen optimalen Flow einstellen lassen. Dabei ist diese rein mechanische Verstellung über die Schaftlänge natürlich nur eine technische Möglichkeit.

Analog ist bei anderen Ventilarten wie Klappventilen oder auch Membranventilen zu verfahren, ohne dass dies hier im einzelnen erläutert werden muss.

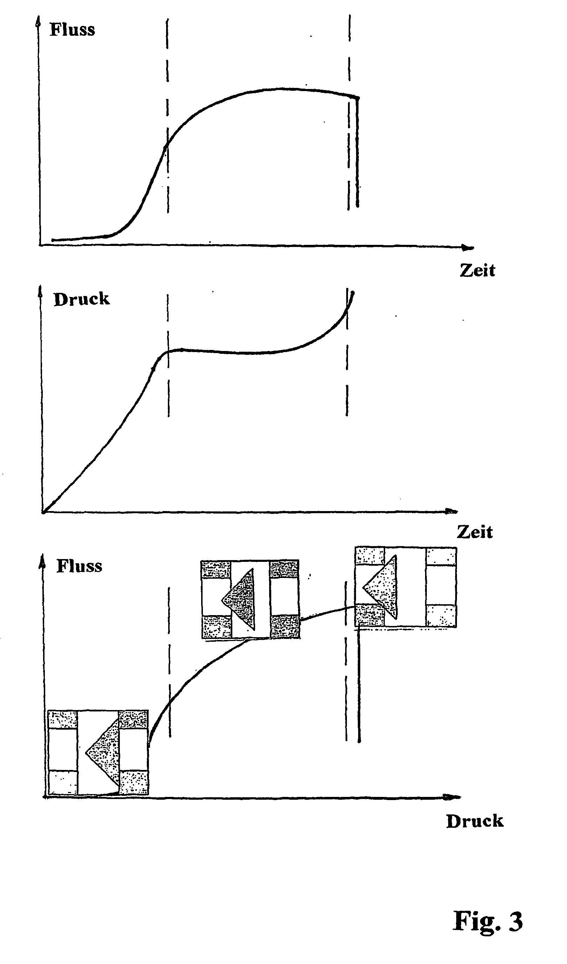

Dieser Mechanismus weist -wie aus den Kennlinien in **Fig. 3** ersichtlich- den Vorteil auf, dass ein Flow nur innerhalb eines bestimmten einstellbaren Bereiches möglich ist. Die Untergrenze wird gesetzt mit dem Öffnungswiderstand zwischen Ventilteller 21 und Ventilsitz 22 und die Obergrenze mit einem Kräftepaar F_{F} << F_{P}, ebenfalls einstellbar. Wird dieser begrenzte Flow mit der Messsensorik abgestimmt, was erfindungsgemäß vorgesehen ist, können Messungen unter optimalen Flowbedingungen realisiert werden.

### Bezugszeichenliste:

- 1: Pneumotachograph
- 2: Mittel zur Druckmessung
- 3: Prozessor
- 4: PEEP-Ventil
- 5: Filter zum Entfernen des Anteils an zu bestimmendem Bestandteil in der Inspirationsluft
- 6: Pneumotachographenrohr
- 7: Sensor
- 8: Öffnung
- 9: optische Kontrolle
- 10: Flowcontroller
- 11: Mundstück
- 12: Bakterienfilter
- 13: Pumpe
- 14: Anschluß
- 15: Sensorkammer
- 16: elektrisches Manometer
- 17: Lamellen
- 18: Klappe
- 19: Peepventilgehäuse
- 20: Einströmöffnung
- 21: Ventilteller
- 22: Ventilsitz
- 23: Ausströmöffnung
- 24: Verschluss, vorzugsweise Kegelform
- 25: Schaft des Kegelverschlusses
- 26: Druckfeder

## Patentansprüche

1. Transportabler Pneumotachograph zur Bestimmung von Bestandteilen des Exspirationsvolumens aufweisend
einen Prozessor (3),
ein ausatemseitig am Pneumotachographen (1) angebrachtes PEEP-Ventil (4), einen einatemseitig am Pneumotachographen (1) angebrachten Filter (5) zum Entfernen des Anteils an zu bestimmendem Bestandteil in der Inspirationsluft, mindestens einen im oder am Pneumotachographenrohr (6) angebrachten Sensor (7), wobei bei einem am Pneumotachographenrohr (6) angeordneten Sensor (7) das Pneumotachographenrohr (6) sensorseitig eine Öffnung (8) aufweist und/oder mindestens ein im Pneumotachographenrohr (6) eingerichtetes Mittel zur Probennahme und ferner
eine optische und/oder akustische Kontrolle (9) des Exspirationsstroms.

2. Transportabler Pneumotachograph nach Anspruch 1, **dadurch gekennzeichnet, dass**
zwischen dem einatemseitig am Pneumotachographen (1) angebrachten Filter (5) und dem Pneumotachographenrohr (6) ein Ventil (18) eingerichtet ist, welches bei der Exspiration die Eintrittsöffnung des Pneumotachographen (1) verschließt.

3. Transportabler Pneumotachograph nach Anspruch 1 oder 2, **dadurch gekennzeichnet, dass**
der Sensor (7) ausgewählt ist aus der Gruppe "elektrochemischer Sensor, Chemilumineszenssensor, NO-Sensor, O₂-Sensor, H₂O₂-Sensor, CO₂-Sensor, CO Sensor, Sensor für Biomarker, insbesondere immunologische Biomarker und/oder molekularbiologische Biomarker" und/oder eine Kombinationssensorik aus den genannten Sensoren ist.

4. Transportabler Pneumotachograph nach einem der Ansprüche 1 bis 3, **dadurch gekennzeichnet, dass**
in der Öffnung (8) zwischen Sensor (7) und Pneumotachographenrohr (6) ein regelbares und/oder ansteuerbares Ventil eingerichtet und dass das Ventil nach der Maßgabe des das Pneumotachographenrohr (6) passierenden Inspirations- oder Exspirations- oder eines Teilexspirationsvolumens den Zustand "offen" oder "geschlossen" hat, wobei vorzugsweise das Ventil durch den Prozessor (3) ansteuerbar ist.

5. Transportabler Pneumotachograph nach einem der Ansprüche 1 bis 4, **dadurch gekennzeichnet, dass**
durch das PEEP-Ventil (4) ein expiratorischer Widerstand, vorzugsweise von 5 bis 20 cm H₂0, einstellbar und erzeugbar ist.

6. Transportabler Pneumotachograph nach einem der Ansprüche 1 bis 5, **dadurch gekennzeichnet, dass**
das PEEP-Ventil (4) abnehmbar ist und eingangsseitig ein federbelastetes Rückschlagventil und ausgangsseitig ein Druckventil aufweist, wobei das federbelastete Rückschlagventil bei Überschreiten eines eingangsseitig erzeugten ersten Drucks öffnet und das Druckventil ausgangsseitig bei Überschreiten eines eingangsseitig erzeugten zweiten Druckes, wobei der zweite Druck größer als der erste Druck ist, schließt.

7. Transportabler Pneumotachograph nach Anspruch 6, **dadurch gekennzeichnet, dass**
das Rückschlagventil aus einem im Peepventilgehäuse (19) vor einer Einströmöffnung (20) mit einem Ventilsitz (22) angeordneten, mit einer Kraft (F_{F}) gegen die Strömungsrichtung im Pneumotachographenrohr (6) gedrückten, den Ventilsitz (22) abdichtenden Ventilteller (21) besteht und
der Ventilteller (21) mit einem Verschluss (24) für die Ausströmöffnung (23) gekoppelt ist, derart, dass ein festlegbares oder einstellbares Maß der Öffnung zwischen Ventilsitz (22) und Ventilteller (21) den vollständigen oder nahezu vollständigen Verschluss der Ausströmöffnung (23) durch den Verschluß (24) bewirkt.

8. Transportabler Pneumotachograph nach Anspruch 6 oder 7, **dadurch gekennzeichnet, dass**
der Verschluss (24) über einen Schaft (25) mit der Rückseite des Ventiltellers (21) verbunden ist.

9. Transportabler Pneumotachograph nach einem der Ansprüche 6 bis 8, **dadurch gekennzeichnet, dass**
die Übertragung des Maßes der Öffnung des Ventiltellers (21) auf die Bewegung des Verschlusses (24) bis zum Verschluss der Ausströmöffnung (23) einstellbar ist.

10. Transportabler Pneumotachograph nach einem der Ansprüche 6 bis 9, **dadurch gekennzeichnet, dass**
der Ventilteller (21) am Ventilsitz (22) vorzugsweise eine der folgenden Anordnungen aufweist
- Klappverschluss, so dass das Öffnen unter einem sich verändernden Winkel zwischen Ventiltellerebene und Ventilsitzebene erfolgt,
- Parallelverschluss, so dass das Öffnen bei Beibehaltung der Parallelität zwischen Ventilteller und Ventilsitzebene erfolgt,
- Membranverschluss, wobei sich die Membran unter PEEP verformt und dabei Öffnungen fireigibt.

11. Transportabler Pneumotachograph nach einem der Ansprüche 6 bis 10, **dadurch gekennzeichnet, dass**
das Peepventilgehäuse (19) eine weitere Luftaustrittsöffnung aufweist, um bei einer vollständig geschlossenen Ausströmöffnung (23) einen Restflow zu gewährleisten.

12. Transportabler Pneumotachograph nach einem der Ansprüche 1 bis 11, **dadurch gekennzeichnet, dass**
die Oberflächen der probeführenden Leitungen oder der Luftführungskanäle des Pneumotachographen derart zur Umsetzung und/oder Bindung von Bestandteilen der Ausatemluft modifiziert sind, dass die Oberfläche selbst physikalisch und/oder chemisch aktiv ist oder auf und/oder in den Oberflächen einzeln oder in Kombination physikalisch und/oder chemisch aktive Membranen, flüssige Filme, poröse Schichten, biologische Immobilisate aufgetragen und/oder eingearbeitet sind.

13. Verfahren zur Bestimmung von Bestandteilen des Exspirationsvolumens mittels eines transportablen Pneumotachographen nach einem der Ansprüche 1 bis 12 aufweisend die Verfahrensschritte
a) Filtern des zu bestimmenden Bestandteils oder der zu bestimmenden Bestandteile aus dem Einatemstrom
b) Messen des Einatemvolumens
c) Ausbildung eines positiven Drucks in der Lunge gegen einen durch ein geschlossenes Druckventil erzeugten Druckwiderstand
d) Öffnen des Druckventils durch Überwinden des Druckwiderstandes
e) Ausbildung eines definierten exspiratorischen Flows auf einem Druckniveau höher als der vorgegebene Druckwiderstand
f) Verwerfen der Totraumluft des Mund- und Rachenraums,
g) Probennahme der exspiratorischen Luft und/oder sensorische Messung des oder der Bestandteile in der exspiratorischen Luft nach Verwerfen der Totraumluft.

14. Verfahren zur Bestimmung von Bestandteilen des Exspirationsvolumens nach Anspruch 13, **dadurch gekennzeichnet, dass**
der zu bestimmende Bestandteil oder die zu bestimmenden Bestandteile ausgewählt sind aus der Gruppe "NO, Stickstoff, Sauerstoff, freie Radikale, CO, H₂0₂ und sonstiger Biomarker".

15. Verfahren zur Bestimmung von Bestandteilen des Exspirationsvolumens nach Anspruch 13 oder 14, **dadurch gekennzeichnet, dass**
ein Teil- und/oder das Gesamtexspirationsvolumen in einem gasundurchlässigen Auffangbehältnis, vorzugsweise einem Gasbeutel, aufgefangen wird.

16. Verfahren zur Bestimmung von Bestandteilen des Exspirationsvolumens nach einem der Ansprüche 13 bis 15, **dadurch gekennzeichnet, dass**
anhand des Einatemvolumens eine Zuordnung von zeitlich nacheinander ausgeatmeten Teilvolumenströme eines Exspirationsvolumens erfolgt, wobei diesen Teilvolumenströme bestimmte Regionen und Zonen des Respirationstraktes zugewiesen werden, und eine Messung von Bestandteilen des Exspirationsvolumens nur dann erfolgt, wenn ein definiertes Teilexspirationsvolumen den Sensor (7) passiert,

17. Verfahren zur Bestimmung von Bestandteilen des Exspirationsvolumens nach einem der Ansprüche 13 bis 16, **dadurch gekennzeichnet, dass**
eine Messung von Bestandteilen des Exspirationsvolumens nur bei Erreichen des oder der Parameter des Exspirationsvolumem "Überwinden des exspiratorischen Widerstands" und/oder "konstanter exspiratorischer Flow" und/oder "Dauer des exspiratorischen Flows" erfolgt.

18. Verfahren zur Bestimmung von Bestandteilen des Exspirationsvolumens nach einem der Ansprüche 13 bis 17, **dadurch gekennzeichnet, dass**
die Werte der Parameter "Überwinden des exspiratorischen Widerstands" und/oder "konstanter exspiratorischen Flow" und/oder "Dauer des exspiratorischen Flows" individuell einstellbar und/oder in Abhängigkeit von der Patientengruppe oder dem Zustand des Exspirationstraktes aus einem Speichermedium, vorzugsweise dem Prozessor (3), abrufbar sind.

## Claims

1. Portable pneumotachograph for measuring components of an expiration volume, and method therefor, having
a processor (3),
a PEEP valve (4) arranged on the pneumotachograph at the expiration side,
a filter (5) arranged on the pneumotachograph at the inspiration side for removal of the fraction of the component being measured in the inspiration air,
at least one sensor (7) arranged in or on the pneumotachograph tube (6), wherein for a sensor (7) arranged on the pneumotachograph tube (6) the pneumotachograph tube (6) has an opening (8) at the sensor side and/or at least one means of taking a sample, disposed in the pneumotachograph tube (6), and moreover
an optical and/or acoustical control (9) of the expiration volume.

2. Portable pneumotachograph according to claim 1, **characterised in that**
a valve (18) is disposed between the filter (5) arranged on the pneumotachograph (1) at the inspiration side and the pneumotachograph tube (6), which closes the inlet opening of the pneumotachograph (1) during the expiration.

3. Portable pneumotachograph according to claim 1 or 2, **characterised in that**
the sensor (7) is chosen from the group of "electrochemical sensors, chemiluminescence sensors, NO sensors, 02 sensors, H2O2 sensors, CO2 sensors, CO sensors, sensors for biomarkers, especially immunological biomarkers and/or molecular biological biomarkers" and/or a combination sensor system of the mentioned sensors.

4. Portable pneumotachograph according to one of claims 1 to 3, **characterised in that**
a regulable and/or actuatable valve is disposed in the opening (8) between sensor (7) and pneumotachograph tube (6) and the valve has the status of "open" or "closed" depending on the inspiration or expiration volume or a partial expiration volume passing through the pneumotachograph tube (6), while preferably the valve is actuatable by the processor (3).

5. Portable pneumotachograph according to one of claims 1 to 4, **characterised in that**
an expiratory resistance of preferably 5 to 20 cm H2O can be adjusted and produced by the PEEP valve (4).

6. Portable pneumotachograph according to one of claims 1 to 5, **characterised in that**
the PEEP valve (4) can be removed and it has a spring-loaded check valve at the inlet side and a pressure valve at the outlet side, wherein the spring-loaded check valve opens upon exceeding a first pressure produced at the inlet side and the pressure valve closes upon exceeding at the outlet side a second pressure produced at the inlet side, where the second pressure is greater than the first pressure.

7. Portable pneumotachograph according to claim 6, **characterised in that**
the check valve consists of a valve disk (21) arranged in the peep valve housing (19) upstream from an inlet opening (20) with a valve seat (22), forced against the flow direction in the pneumotachograph tube (6) with a force (F_{F}) and sealing off the valve seat (22), and
the valve disk (21) is coupled to a closure (24) for the outlet opening (23), such that a determinable or adjustable degree of opening between valve seat (22) and valve disk (21) brings about the total or nearly total closure of the outlet opening (23) by the closure (24).

8. Portable pneumotachograph according to claim 6 or 7, **characterised in that**
the closure (24) is connected by a shaft (25) to the back side of the valve disk (21).

9. Portable pneumotachograph according to one of claims 6 to 8, **characterised in that**
the transmission of the degree of opening of the valve disk (21) to the movement of the closure (24) until the outlet opening (23) is closed is adjustable.

10. Portable pneumotachograph according to one of claims 6 to 9, **characterised in that**
the valve disk (21) has one of the following arrangements at the valve seat (22)
- flap closure, so that the opening occurs at a varying angle between the plane of the valve disk and the plane of the valve seat,
- parallel closure, so that the opening occurs while maintaining parallelness between valve disk and valve seat plane,
- membrane closure, wherein the membrane is deformed under PEEP and thereupon clears openings.

11. Portable pneumotachograph according to one of claims 6 to 10, **characterised in that**
the peep valve housing (19) has an additional air outlet opening to ensure a residual flow when the outlet opening (23) is completely closed.

12. Portable pneumotachograph according to one of claims 1 to 11, **characterised in that**
the surfaces of the lines carrying the sample or the air channels of the pneumotachograph are modified for the conversion and/or binding of components of the expired air such that the surface itself is physically and/or chemically active, or physically and/or chemically active membranes, liquid films, porous coatings, biologically immobilised components are deposited and/or worked in, individually or in combination, on and/or in the surfaces.

13. Method for measuring of components of the expiration volume by means of a portable pneumotachograph according to one of claims 1 to 12, having the method steps of
a) filtering the component being measured or the components being measured from the inspiration flow
b) measuring the inspiration volume
c) forming a positive pressure in the lungs against a pressure resistance produced by a closed pressure valve
d) opening of the pressure valve by overcoming the pressure resistance
e) forming a defined expiratory flow at a pressure level higher than the predetermined pressure resistance
f) rejecting the dead-space air of the mouth and throat space,
g) taking a sample of the expiratory air and/or sensor measurement of the component or components in the expiratory air after rejecting the dead-space air.

14. Method for measuring of components of the expiration volume according to claim 13, **characterised in that**
the component being measured or the components being measured are chosen from the group of "NO, nitrogen, oxygen, free radicals, CO, H2O2 and other biomarkers".

15. Method for measuring of components of the expiration volume according to claim 13 or 14, **characterised in that**
a partial and/or the total expiration volume is captured in a gas-impermeable trapping volume, preferably a gas bag.

16. Method for measuring of components of the expiration volume according to one of claims 13 to 15, **characterised in that**
the inspiration volume is used to co-ordinate partial volume flows of an expiration volume that are breathed out consecutively in time, these partial volume flows being assigned certain regions and zones of the respiratory tract, and a measurement of components of the expiration volume is done only when a defined partial expiration volume has passed the sensor (7).

17. Method for measuring of components of the expiration volume according to one of claims 13 to 16, **characterised in that**
a measurement of components of the expiration volume is done only upon reaching the parameter or parameters of the expiration volume: "overcoming of the expiratory resistance" and/or "constant expiratory flow" and/or "duration of the expiratory flow".

18. Method for measuring of components of the expiration volume according to one of claims 13 to 17, **characterised in that**
the values of the parameters: "overcoming of the expiratory resistance" and/or "constant expiratory flow" and/or "duration of the expiratory flow" are individually adjustable and/or can be called up in dependence on the patient group or the condition of the expiratory tract from a storage medium, preferably the processor (3).

## Revendications

1. Pneumotachographe transportable pour mesurer des composants du volume d'expiration, présentant
un processeur (3),
une valve PEEP (4) installée côté expiration sur le pneumotachographe (1), un filtre (5) installé côté inspiration sur le pneumotachographe (1) pour éliminer la fraction du composant à mesurer contenue dans l'air inspiré,
au moins un détecteur (7) installé dans ou sur le tube de pneumotachographe (6), ledit tube de pneumotachographe (6), lorsque le détecteur (7) est installé sur le tube de pneumotachographe (6), présentant côté détecteur une ouverture (8) et/ou au moins un moyen de prélèvement d'échantillons disposé dans le tube de pneumotachographe (6), ainsi que
un contrôle visuel et/ou sonore (9) du flux d'aspiration.

2. Pneumotachographe transportable selon la revendication 1, **caractérisé en ce qu'**une valve (18) qui ferme l'orifice d'entrée du pneumotachographe (1) lors de l'expiration est disposée entre le filtre (5) installé côté inspiration sur le pneumotachographe (1) et le tube de pneumotachographe (6).

3. Pneumotachographe transportable selon la revendication 1 ou la revendication 2, **caractérisé en ce que** le détecteur (7) est choisi dans le groupe "détecteur électrochimique, détecteur de chimiluminescence, détecteur de NO, détecteur d'O₂, détecteur de H₂O₂, détecteur de CO₂, détecteur de CO, détecteur de biomarqueurs, en particulier de biomarqueurs immunologiques et/ou de biomarqueurs de biologie moléculaire", et/ou un système de détection combiné constitué des détecteurs précités.

4. Pneumotachographe transportable selon l'une des revendications 1 à 3, **caractérisé en ce qu'**une valve réglable et/ou commandable est disposée dans l'ouverture (8) entre le détecteur (7) et le tube de pneumotachographe (6) et **en ce que** ladite valve, en fonction du volume d'inspiration ou expiration ou d'un volume d'expiration partiel qui passe dans le tube de pneumotachographe (6), se trouve à l'état "ouvert" ou "fermé", la valve étant de préférence commandable par le processeur (3).

5. Pneumotachographe transportable selon l'une des revendications 1 à 4, **caractérisé en ce que** la valve PEEP (4) permet de générer et ajuster une résistance expiratoire, de préférence dans la plage de 5 à 20 cm H₂O.

6. Pneumotachographe transportable selon l'une des revendications 1 à 5, **caractérisé en ce que** la valve PEEP (4) est amovible et présente côté entrée un clapet antiretour chargé par ressort et côté sortie un clapet régulateur de pression, le clapet antiretour chargé par ressort s'ouvrant en cas de dépassement d'une première pression générée côté entrée et le clapet régulateur de pression côté sortie se fermant en cas de dépassement d'une deuxième pression générée côté entrée, laquelle deuxième pression est supérieure à la première pression.

7. Pneumotachographe transportable selon la revendication 6, **caractérisé en ce que** le clapet antiretour est constitué d'une tête de clapet (21) disposée dans le boîtier de valve PEEP (19) devant un orifice d'entrée (20) comportant un siège de clapet (22), comprimée avec une force (F_{F}) contre le sens d'écoulement dans le tube de pneumotachographe (6) et assurant l'étanchéité du siège de clapet (22), et **en ce que** la tête de clapet (21) est couplée à un obturateur (24) de l'orifice de sortie (23) de façon qu'une cote définissable ou réglable de l'ouverture entre le siège de clapet (22) et la tête de clapet (21) provoque l'obturation complète ou presque complète de l'orifice de sortie (23) par l'obturateur (24).

8. Pneumotachographe transportable selon la revendication 6 ou la revendication 7, **caractérisé en ce que** l'obturateur (24) est relié par l'intermédiaire d'une tige (25) à l'arrière de la tête de clapet (21).

9. Pneumotachographe transportable selon l'une des revendications 6 à 8, **caractérisé en ce que** la transmission de la cote d'ouverture de la tête de clapet (21) au mouvement de l'obturateur (24) jusqu'à l'obturation de l'orifice de sortie (23) est réglable.

10. Pneumotachographe transportable selon l'une des revendications 6 à 9, **caractérisé en ce que** la tête de clapet (21) présente de préférence une des dispositions suivantes sur le siège de clapet (22) :
- Obturateur basculant, de sorte que l'ouverture se fait selon un angle variable entre le plan de la tête de clapet et le plan du siège de clapet,
- Obturateur parallèle, de sorte que l'ouverture se fait en conservant le parallélisme entre la tête de clapet et le siège de clapet,
- Obturateur à membrane, la membrane se déformant sous l'effet de la PEEP et dégageant ainsi des ouvertures.

11. Pneumotachographe transportable selon l'une des revendications 6 à 10, **caractérisé en ce que** le boîtier de valve PEEP (19) présente un autre orifice de sortie d'air afin de garantir un débit résiduel lorsque l'orifice de sortie (23) est complètement fermé.

12. Pneumotachographe transportable selon l'une des revendications 1 à 11, **caractérisé en ce que** les surfaces des lignes véhiculant l'échantillon ou des conduits véhiculant l'air du pneumotachographe sont modifiées en vue de la réaction et/ou de la liaison de composants de l'air expiré de telle façon que la surface elle-même est physiquement et/ou chimiquement active ou que des membranes, des films liquides, des couches poreuses, des immobilisats biologiques ayant une activité physique et/ou chimique sont déposés et/ou incorporés seuls ou en combinaison sur et/ou dans les surfaces.

13. Procédé de mesure de composants du volume d'expiration au moyen d'un pneumotachographe transportable selon l'une des revendications 1 à 12, comprenant les étapes consistant à
a) éliminer par filtration le ou les composants à mesurer du flux d'inspiration,
b) mesurer le volume inspiré,
c) établir une pression positive dans le poumon contre une résistance à la pression générée par un clapet régulateur de pression fermé,
d) ouvrir le clapet régulateur de pression en surmontant la résistance à la pression,
e) former un flux expiratoire défini à un niveau de pression supérieur à la résistance à la pression prédéfinie,
f) éliminer l'air de l'espace mort de la zone de la bouche et de la gorge,
g) prélever un échantillon de l'air expiré et/ou mesurer au moyen de détecteurs le ou les composants présents dans l'air expiré après avoir éliminé l'air de l'espace mort.

14. Procédé de mesure de composants du volume d'expiration selon la revendication 13, **caractérisé en ce que** le ou les composants à mesurer sont choisis dans le groupe "NO, azote, oxygène, radicaux libres, CO, H₂O₂ et autres biomarqueurs".

15. Procédé de mesure de composants du volume d'expiration selon la revendication 13 ou la revendication 14, **caractérisé en ce qu'**une partie et/ou la totalité du volume d'expiration est collectée dans un récipient de collecte étanche aux gaz, de préférence dans une poche à gaz.

16. Procédé de mesure de composants du volume d'expiration selon l'une des revendications 13 à 15, **caractérisé en ce que**, à partir du volume inspiré, on effectue une affectation de débits volumétriques partiels d'un volume d'expiration expirés successivement dans le temps, ces débits volumétriques partiels étant associés à des régions et des zones déterminées du système respiratoire, et on effectue ensuite une mesure de composants du volume d'expiration seulement lorsqu'un débit volumétrique partiel défini passe dans le détecteur (7).

17. Procédé de mesure de composants du volume d'expiration selon l'une des revendications 13 à 16, **caractérisé en ce qu'**on effectue une mesure de composants du volume d'expiration seulement lorsque le ou les paramètres du volume d'expiration "surmonter la résistance expiratoire" et/ou "débit expiratoire constant" et/ou "durée du débit expiratoire" sont atteints.

18. Procédé de mesure de composants du volume d'expiration selon l'une des revendications 13 à 17, **caractérisé en ce que** les valeurs des paramètres "surmonter la résistance expiratoire" et/ou "débit expiratoire constant" et/ou "durée du débit expiratoire" sont réglables individuellement et/ou appelables en fonction du groupe de patients ou de l'état du tractus expiratoire à partir d'un support de stockage, de préférence le processeur (3).
